# EUROPEAN PATENT APPLICATION

(11) **EP 4 756 820 A1**
(43) Date of publication of application: **10.06.2026**
(21) Application number: 23948572.5
(22) Date of filing: 14.08.2023
(51) Int. Cl.: G16H 20/10, G16H 10/60, G16H 70/40, G16H 50/20, G16H 50/70

(54) **METHOD FOR MANAGING DRUG PRESCRIPTION AND DOSAGE BASED ON DIGITAL BIOMARKER AND APPARATUS FOR PERFORMING SAME METHOD**

(30) Priority: 04.08.2023 KR 20230102204
(71) Applicant: WELT Corp., Ltd, Seocho-gu Seoul 06628 (KR)
(72) Inventor: KANG, Seong Ji, Seoul 06366 (KR); ROH, Hye Kang, Seoul 05507 (KR); KIM, Joo Young, Seoul 04014 (KR); LEE, Do Hyun, Yongin-si Gyeonggi-do 16826 (KR); JEONG, Hwa Young, Incheon 21451 (KR)
(74) Representative: AWA Sweden AB
(86) International application number: PCT/KR2023/012013
(87) International publication number: WO 2025/033577

(57) **Abstract**

The present invention relates to a digital biomarker-based prescription management method and an apparatus for performing the method. The digital biomarker-based prescription management method may comprise the steps of: receiving, by a prescription management device, user data; and generating, by the prescription management device, prescription data on the basis of the user data. The present invention is a technology developed through the Seoul Business Agency's 2021 Bio-Medical Technology Commercialization Support Project (BT210029) "Technology Development of Precision Digital Therapeutic Agent for Alcohol Addiction."

## Description

### BACKGROUND

### 1. Field of the Invention

The present invention relates to a method of managing a medication prescription and dosage on the basis of a biomarker and an apparatus for performing the same. More specifically, the present invention relates to a method of managing a medication prescription and dosage on the basis of a biomarker to make a prescription adaptively to a user on the basis of user data including a user biomarker and an apparatus for performing the same.

### 2. Discussion of Related Art

With the development of various smart technologies, data of personal daily activities is recorded, and individual life can be efficiently managed on the basis of the recorded data. In the meantime, health-related data logging is attracting attention due to the increasing interest in healthcare. Many users have already been generating and utilizing various health-related data including data on exercise, diet, sleep, and the like through user devices such as smartphones, wearable devices, and the like. In the past, health-related data was generated and managed only by medical institutions, but now users have begun to generate and manage their own health-related data through user devices such as smartphones and wearable devices.

In many cases, health-related data logging is performed through a wearable device. A wearable device is a user device that is carried by or attached to a user. Due to the development of Internet of things (IoT) and the like, wearable devices are frequently used for collecting health-related data. A wearable device may collect a user's physical change information and surrounding data of the user through equipment and provide advice required for the user's healthcare on the basis of the collected data.

A user's health-related data may include a user biomarker, and research is ongoing on a method of making a medical prescription adaptively to a user on the basis of the user's health-related data.

The present application was developed through the bio-medical technology commercialization support project (BT210029) "Development of Precise Digital Therapeutics for Alcoholism" by Seoul Business Agency in 2021.

As related art, there is Korean Patent No. 10-2425479.

### SUMMARY OF THE INVENTION

The present invention is directed to solving all the foregoing problems.

The present invention is also directed to predicting effects and side effects of a prescription on the basis of artificial intelligence and generating prescription data adaptively to a user.

The present invention is also directed to predicting effects and side effects of a prescription in consideration of user data through artificial intelligence engine tuning optimized for the user.

A representative configuration of the present invention for achieving the above objects is as follows.

According to an aspect of the present invention, there is provided a method of managing a prescription on the basis of a digital biomarker comprises receiving, by a prescription management device, user data and generating, by the prescription management device, prescription data on the basis of the user data.

Meanwhile, the prescription management device generates the prescription data on the basis of an artificial intelligence engine, the artificial intelligence engine determines prescription effects and prescription side effects on the basis of the user data and generates the prescription data on the basis of the prescription effects and the prescription side effects.

Further, the artificial intelligence engine includes a default artificial intelligence engine and a user-optimized artificial intelligence engine, the user-optimized artificial intelligence engine is obtained by tuning the default artificial intelligence engine using the user data.

According to another aspect of the present invention, there is provided a prescription management apparatus for managing a prescription on the basis of a digital biomarker performs the operations of: receiving user data and generating prescription data on the basis of the user data.

Meanwhile, the prescription management device generates the prescription data on the basis of an artificial intelligence engine, the artificial intelligence engine determines prescription effects and prescription side effects on the basis of the user data and generates the prescription data on the basis of the prescription effects and the prescription side effects.

Further, the artificial intelligence engine includes a default artificial intelligence engine and a user-optimized artificial intelligence engine, the user-optimized artificial intelligence engine is obtained by tuning the default artificial intelligence engine using the user data.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG. 1 is a block diagram of a system for managing a prescription on the basis of a digital biomarker according to an exemplary embodiment of the present invention.
FIG. 2 is a conceptual diagram illustrating operations of a prescription management device according to an exemplary embodiment of the present invention.
FIGS. 3 and 4 are conceptual diagrams illustrating specialization of an artificial intelligence engine according to an exemplary embodiment of the present invention.
FIG. 5 is a conceptual diagram of an artificial intelligence engine for generating prescription data for a user according to an exemplary embodiment of the present invention.
FIG. 6 is a conceptual diagram illustrating a method of generating a user-optimized artificial intelligence engine according to an exemplary embodiment of the present invention.
FIG. 7 is a conceptual diagram illustrating a method of generating a user-optimized artificial intelligence engine according to an exemplary embodiment of the present invention.

### DETAILED DESCRIPTION OF EXEMPLARY EMBODIMENTS

The detailed description of the present invention will be made with reference to the accompanying drawings showing examples of specific embodiments of the present invention. These embodiments will be described in detail such that the present invention can be performed by those skilled in the art. It should be understood that various embodiments of the present invention are different but are not necessarily mutually exclusive. For example, a specific shape, structure, and characteristic of an embodiment described herein may be implemented in another embodiment without departing from the scope and spirit of the present invention. In addition, it should be understood that a position or arrangement of each component in each disclosed embodiment may be changed without departing from the scope and spirit of the present invention. Accordingly, there is no intent to limit the present invention to the detailed description to be described below. The scope of the present invention is defined by the appended claims and encompasses all equivalents that fall within the scope of the appended claims. Like reference numerals refer to the same or like elements throughout the description of the figures.

Hereinafter, in order to enable those skilled in the art to practice the present invention, exemplary embodiments of the present invention will be described in detail with reference to the accompanying drawings.

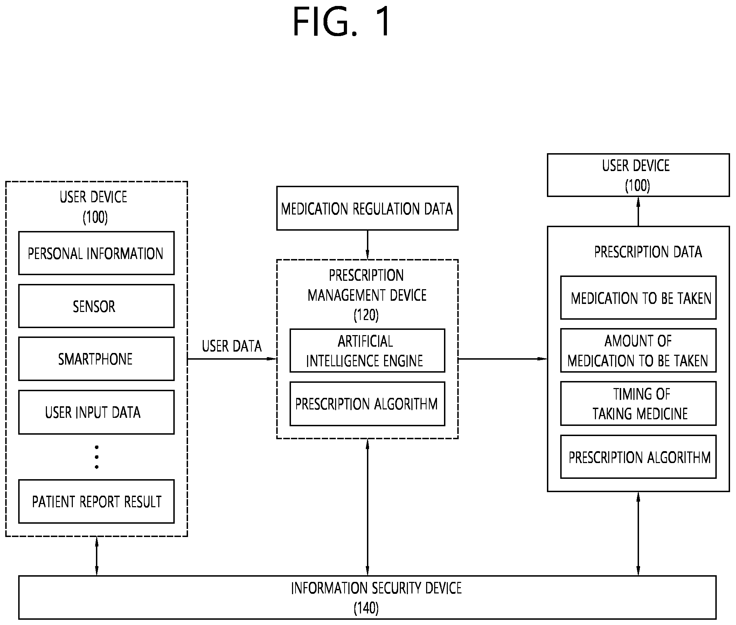

FIG. 1 is a block diagram of a system for managing a prescription on the basis of a digital biomarker according to an exemplary embodiment of the present invention.

FIG. 1 shows a method of making a prescription adaptively to each user on the basis of user data.

Referring to FIG. 1, the system for managing a prescription on the basis of a digital biomarker may include a user device 100, a prescription management device 120, and an information security device 140.

The user device 100 may generate user data for a prescription for a user, transmit the user data to the prescription management device 120, and receive prescription data which is determined adaptively to the user.

The user data may include various data used for a prescription for the user. For example, the user data may include biomarker data acquired through the user device 100 (e.g., a smartphone or a wearable device), input data separately input by the user, and data of the user's personal information (sex, age, and the like).

The prescription data may include data prescribed for the user by the prescription management device 120. The prescription management device 120 may generate prescription data on the basis of the user data and transmit the prescription data to the user device 100.

The prescription data may include information on a medication to be taken, the amount of the medication to be taken, and the timing of taking the medication. The amount of a general medication to be taken by the user and the timing of taking the medication may be included in the prescription data. Also, the prescription data may include a prescription algorithm based on digital therapeutics. For example, when there are digital therapeutics for treating insomnia or an eating disorder, a prescription for the user based on the digital therapeutics may also be included in the user prescription data.

The prescription management device 120 may determine the prescription data for the user on the basis of the user data. For example, the prescription management device 120 may be an artificial intelligence engine and generate optimal prescription data for the user on the basis of the user data. The artificial intelligence engine may learn changes in users caused by prescriptions for the users on the basis of user data resulting from prescription data and give a prescription adaptive to each user. The prescription management device 120 may include a plurality of artificial intelligence engines. Each of the artificial intelligence engines may generate different data for a user prescription (e.g., a predicted value of a prescription effect, a predicted value of a prescription side effect, a medication to be taken, the amount of the medication to be taken, the timing of taking the medication, a prescription algorithm, prescription data, and the like), and prescription data may be generated on the basis of the generated data. Also, the prescription management device 120 may generate prescription data on the basis of a prescription algorithm other than artificial intelligence.

According to an exemplary embodiment of the present invention, one or more of the artificial intelligence engines included in the prescription management device 120 may be classified as a default artificial intelligence engine not yet specialized for a user and a user-optimized artificial intelligence engine specialized for a user.

The default artificial intelligence engine is an artificial intelligence engine used before optimal tuning for a user and may generate default prescription data. The user-optimized artificial intelligence engine is an artificial intelligence engine used after optimal tuning for a user on the basis of user data and may generate optimized prescription data.

The default artificial intelligence engine is additionally trained using user data so that a user-optimized artificial intelligence engine is generated, and the user-optimized artificial intelligence engine may generate optimized prescription data.

Also, the prescription management device 120 may generate prescription data on the basis of medication regulation data. The medication regulation data may be learned by another artificial intelligence engine or implemented as another algorithm so that prescription data does not violate medication regulations.

The information security device 140 may be implemented for the security of information generated by the user device 100 and the prescription management device 120.

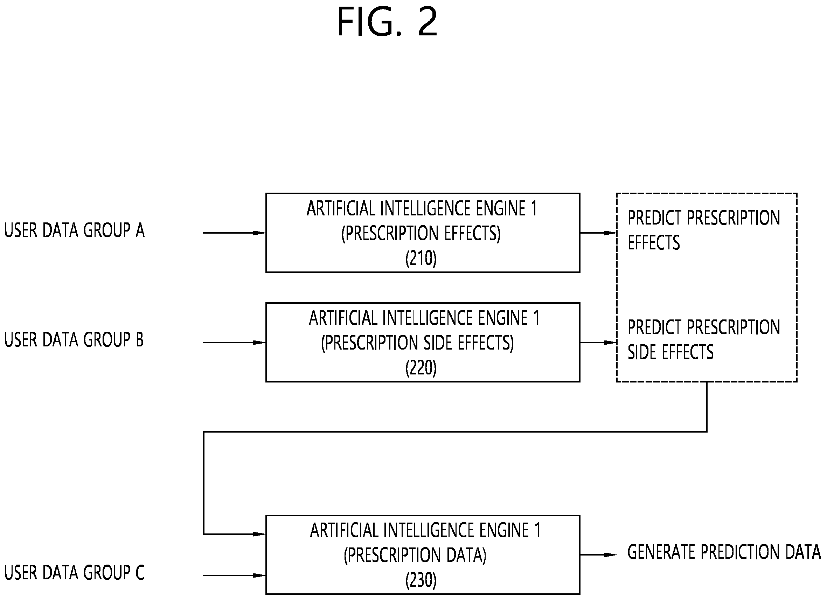

FIG. 2 is a conceptual diagram illustrating operations of a prescription management device according to an exemplary embodiment of the present invention.

FIG. 2 shows a method for a prescription management device to generate prescription data on the basis of artificial intelligence engines.

Referring to FIG. 2, a prescription management device may include a plurality of artificial intelligence engines, and the plurality of artificial intelligence engines may generate a plurality of pieces of sub-prescription data. Prescription data may be generated on the basis of the plurality of pieces of sub-prescription data.

Although user data may be received not only from a user device but also from various other devices, it is assumed for convenience of description that user data generated by a user device is transmitted to the prescription management device.

User data may be preprocessed to be transmitted to each of the plurality of artificial intelligence engines. For example, a plurality of pieces of user data having various formats may be grouped into a plurality of user data groups, and the plurality of user data groups may be separately transmitted to the plurality of artificial intelligence engines. The plurality of artificial intelligence engines may separately generate a plurality of pieces of sub-prescription data.

Prescription data to be transmitted to a user may be generated on the basis of the plurality of pieces of sub-prescription data.

For example, artificial intelligence engine 1 (210) may be implemented to predict prescription effects.

Artificial intelligence engine 2 (220) may be implemented to predict prescription side effects. Artificial intelligence engine 3 (230) may be implemented to determine prescription data (a type of medication to be taken, the amount of the medication to be taken, the timing of taking the medication, and prescription data based on a digital therapeutics prescription algorithm).

User data group 1 to user data group 3 may be defined to correspond to artificial intelligence engine 1 to artificial intelligence engine 3, respectively. User data group 1 to user data group 3 generated on the basis of user data may be transmitted to artificial intelligence engine 1 to artificial intelligence engine 3, respectively.

Also, according to an exemplary embodiment of the present invention, a specific artificial intelligence engine may receive an output value generated by another artificial intelligence engine as an input value. For example, prescription data generated by artificial intelligence engine 3 (230) may be generated when artificial intelligence engine 3 (230) receives not only a user data group but also prescription effect prediction data and prescription side effect prediction data generated by the other artificial intelligence engines.

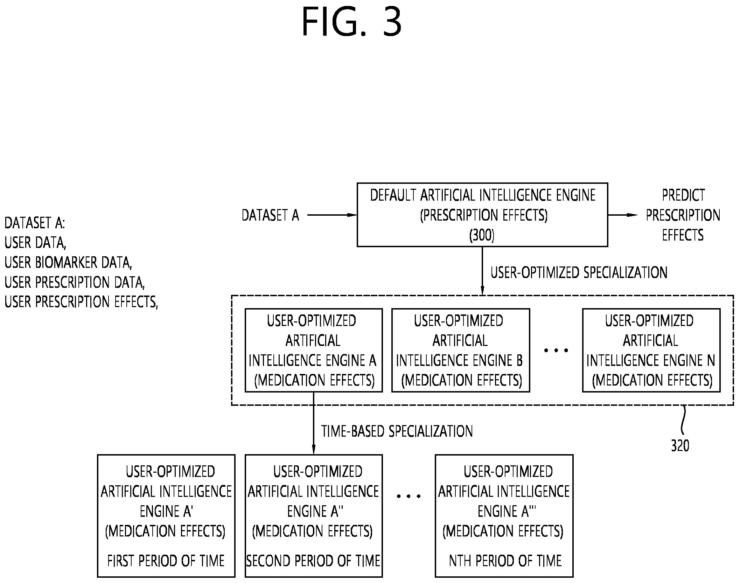

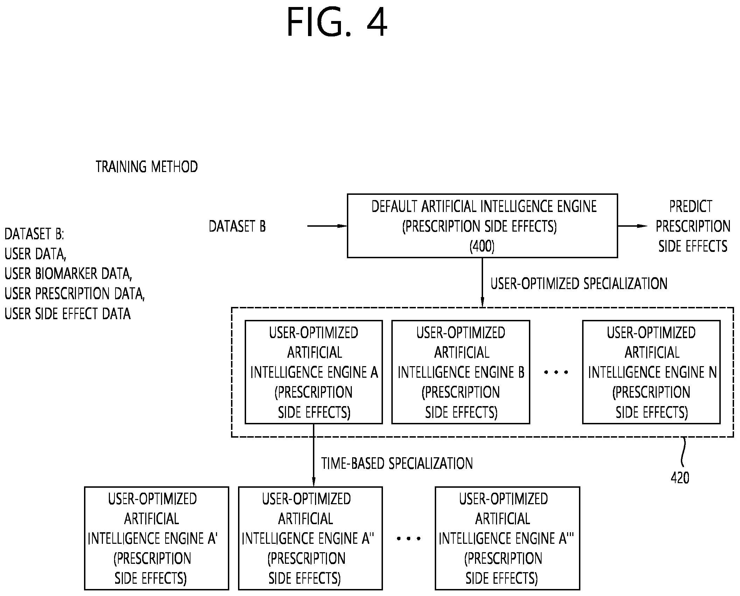

FIGS. 3 and 4 are conceptual diagrams illustrating specialization of an artificial intelligence engine according to an exemplary embodiment of the present invention.

FIGS. 3 and 4 show processes in which an artificial intelligence engine is specialized on the basis of user data.

Referring to FIG. 3, a default artificial intelligence engine (prescription effects) 300 for predicting prescription effects may be tuned as a user-optimized artificial intelligence engine (prescription effects) 320 on the basis of user data.

The default artificial intelligence engine (prescription effects) 300 may be an artificial intelligence engine for predicting effects of medication prescribed for a user or effects of a prescription based on a digital treatment algorithm for the user. The default artificial intelligence engine (prescription effects) 300 may be an artificial intelligence engine not yet optimized for a user on the basis of user data. The user-optimized artificial intelligence engine (prescription effects) 320 is an artificial intelligence engine optimized for a user on the basis of user data.

For example, the default artificial intelligence engine (prescription effects) 300 may be tuned on the basis of dataset A so that the user-optimized artificial intelligence engine (prescription effects) 320 may be generated. Dataset A may include data for optimizing a prescription for a user in consideration of the user's characteristics such as prescription data for the user, user biomarker data, user prescription effect data, and the like. The default artificial intelligence engine (prescription effects) 300 may be specialized on the basis of dataset A of the user so that the user-optimized artificial intelligence engine (prescription effects) 320 may be generated.

The default artificial intelligence engine (prescription effects) 300 may also be specialized as user-optimized artificial intelligence engines (prescription effects) 320 for user groups which are determined by grouping a plurality of users having different prescription effects by prescription effect. Alternatively, the default artificial intelligence engine (prescription effects) 300 may be specialized as a user-optimized artificial intelligence engine (prescription effects) 320 in which parameter adjustments only for a user are made.

The user-optimized artificial intelligence engine (prescription effects) 320 may generate a medication effect prediction value for a user by time. Alternatively, the default artificial intelligence engine (prescription effects) 300 may be specialized on the basis of time so that user-optimized artificial intelligence engines (prescription effects) 320 defined for time-specific prescription effects by time may be generated.

The default artificial intelligence engine (prescription effects) 300 and the user-optimized artificial intelligence engine (prescription effects) 320 may generate predicted prescription effects for a plurality of prescriptions. For example, when prescription A, prescription B, and prescription C are simultaneously made, predicted prescription effects according to prescription time points of prescription A, prescription B, and prescription C may be generated and provided.

Referring to FIG. 4, a default artificial intelligence engine (prescription side effects) 400 may be specialized on the basis of user data so that a user-optimized artificial intelligence engine (prescription side effects) 420 may be generated.

The default artificial intelligence engine (prescription side effects) 400 may be tuned on the basis of dataset B so that the user-optimized artificial intelligence engine (prescription side effects) 420 may be generated. Dataset B may include user prescription data, user biomarker data, user prescription side effect data, and the like. The default artificial intelligence engine (prescription side effects) 400 may be specialized on the basis of dataset B of the user so that the user-optimized artificial intelligence engine (prescription side effects) 420 may be generated.

User-optimized artificial intelligence engines (prescription side effects) 420 may be generated for users or user groups or generated by time.

The default artificial intelligence engine (prescription side effects) 400 and the user-optimized artificial intelligence engine (prescription side effects) 420 may generate predicted prescription side effects for a plurality of prescriptions. For example, when prescription A, prescription B, and prescription C are simultaneously made, predicted prescription side effects according to prescription time points of prescription A, prescription B, and prescription C may be generated and provided.

A default artificial intelligence engine may be generated on the basis of user data of all users, and a user-optimized artificial intelligence engine may be generated by optimally specializing the default artificial intelligence engine on the basis of user data of a specific user or a specific user group.

For convenience of description, it has been assumed that there are artificial intelligence engines for prescription effects and prescription side effects. However, other artificial intelligence engines may also operate as a default artificial intelligence engine and user-optimized artificial intelligence engines obtained by specializing a default artificial intelligence engine.

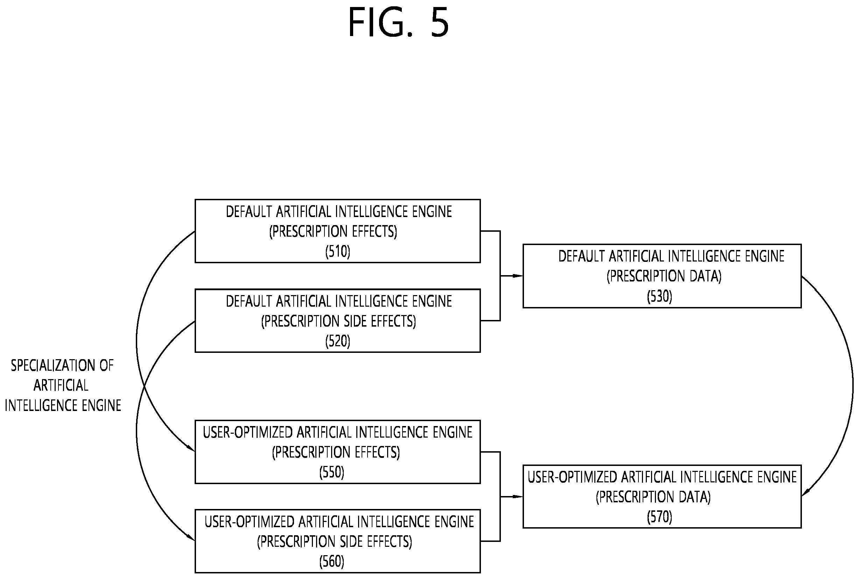

FIG. 5 is a conceptual diagram of an artificial intelligence engine for generating prescription data for a user according to an exemplary embodiment of the present invention.

FIG. 5 illustrates a method of generating an artificial intelligence engine (prescription data) for generating prescription data for a user.

Referring to FIG. 5, an artificial intelligence engine (prescription data) may be generated through training with data based on other artificial intelligence engines such as an artificial intelligence engine (prescription effects), an artificial intelligence engine (prescription side effects), and the like.

The default artificial intelligence engine (prescription data) 530 may generate prescription data for a user on the basis of predicted prescription effect data generated by a default artificial intelligence engine (prescription effects) 510 and predicted prescription side effect data generated by a default artificial intelligence engine (prescription side effects) 520. The prescription data may include a medication to be taken, the amount of the medication to be taken, the timing of taking the medication, a digital therapeutics prescription algorithm, digital therapeutics prescription data, and the like.

In other words, the default artificial intelligence engine (prescription data) 530 may interoperate with the default artificial intelligence engine (prescription effects) 510 and the default artificial intelligence engine (prescription side effects) 520 to generate prescription data on the basis of output values from the default artificial intelligence engine (prescription effects) 510 and the default artificial intelligence engine (prescription side effects) 520.

Also, the default artificial intelligence engine (prescription data) 530 may learn medication regulation data and output prescription data which satisfies the medication regulation data. For example, the medication regulation data may be learned through feedback of the output prescription data, and the default artificial intelligence engine (prescription data) 530 may output prescription data suited for the medication regulation data.

The default artificial intelligence engine (prescription data) 530 may interoperate with the default artificial intelligence engine (prescription effects) 510 and the default artificial intelligence engine (prescription side effects) 520 to generate prescription data in consideration of effects of a plurality of prescriptions and side effects of the plurality of prescriptions.

When a user-optimized artificial intelligence engine (prescription effects) 550 and/or a user-optimized artificial intelligence engine (prescription side effects) 560 is obtained by specializing the default artificial intelligence engine (prescription effects) 510 and/or the default artificial intelligence engine (prescription side effects) 520, the default artificial intelligence engine (prescription data) 530 connected to the user-optimized artificial intelligence engine (prescription effects) 550 and/or the user-optimized artificial intelligence engine (prescription side effects) 560 may be changed into a user-optimized artificial intelligence engine (prescription data) 570.

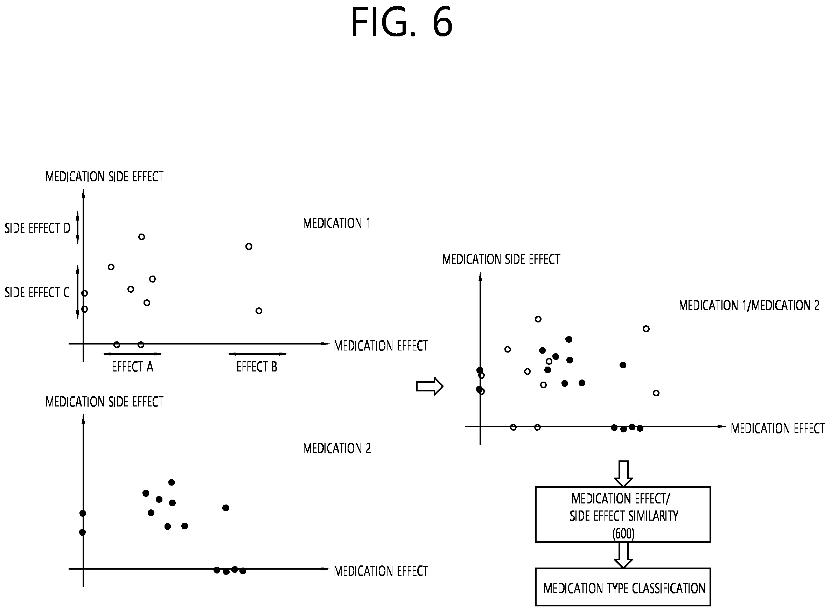

FIG. 6 is a conceptual diagram illustrating a method of generating a user-optimized artificial intelligence engine according to an exemplary embodiment of the present invention.

FIG. 6 shows a method of generating prescription data by generating a user-optimized artificial intelligence engine for each type of medication.

Referring to FIG. 6, user-optimized artificial intelligence engines may be generated for types of medications. For example, prescription effects and prescription side effects may be determined for types of medications defined for each medication attribute, and prescription data may be generated for each type of medication.

To determine a type of medication, a primary type classification may be performed on the basis of medication constituents constituting a medication. Among a plurality of medication constituents constituting a medication, medication constituents (effects) related to effects may be extracted. Also, among a plurality of medication constituents constituting a medication, medication constituents (side effects) related to effects may be extracted.

A medication type (effects) based on effects may be determined on the basis of the medication constituents (effects), and a medication type (side effects) based on side effects may be determined on the basis of the medication constituents (side effects). A type of medication may be determined on the basis of the medication type (effects) and the medication type (side effects). The medication type (effects) and the medication type (side effects) may be embedded in two dimensions.

For example, a medication type (effects) may be embedded on the basis of a first-dimension value which is set according to effects on the basis of a first dimension. The medication type (effects) may be embedded so that similar effects may have first-dimension values close to each other. The medication type (side effects) may be embedded on the basis of a second-dimension value which is set according to side effects on the basis of a second dimension. The medication type (side effects) may be embedded so that similar side effects may have second-dimension values close to each other. More specifically, effect A may be positioned between x1 and x2 in the first dimension according to the degree of effect A. In the first dimension, effect B may be positioned between x3 and x4 according to the degree of effect B. As another example, in the second dimension, side effect C may be positioned between y1 and y2 according to the degree of side effect C. In the second dimension, side effect D may be positioned between y3 and y4 according to the degree of side effect D.

One medication may have a plurality of different medication effects and medication side effects, and one medication may have a plurality of individual embedded values. A medication type may be determined on the basis of a plurality of individual embedded values set for each medication. A medication effect/side effect similarity 600 may be determined on the basis of similarities between a plurality of individual embedded values (e.g., distances between the individual embedded values), and medications having the same medication effects and the same medication side effects on the basis of the medication effect/side effect similarity 600 may be classified into the same medication type. Here, the medication type may be determined on the basis of the plurality of individual embedded values except for some in consideration of the degree of effects and the degree of side effects.

Also, weights may be adjusted in each dimension so that medications having relatively strong medication effects or relatively strong medication side effects may be classified into the same medication type in consideration of medication effects and medication side effects. In addition, relatively high weights are set for relatively important medication effects and relatively fatal side effects so that medications having relatively important medication effects which are similar to each other and similar probabilities of relatively fatal side effects may be classified into the same medication type.

In the present invention, a default artificial intelligence engine may be generated in consideration of a medication type determined on the basis of individual embedded values as described above.

The default artificial intelligence engine may be specialized on the basis of user data. Here, user groups may be made on the basis of user data, and the default artificial intelligence engine may be specialized as user-optimized artificial intelligence engines.

A user group may be set for each medication type in consideration of actual prescription effects and prescription side effects on users and relationships between user data. When a prescription effect for a specific user data value is a threshold effect or more on the basis of user data or a prescription side effect for the specific user data value is a threshold side effect or more, a user having the user data value may be set as a user group, and a user-optimized artificial intelligence engine may be specialized for the user group.

In this way, with regard to a new medication, prescription effects and prescription side effects may be predicted for each user in consideration of individual embedded values based on medication constituents (effects) and medication constituents (side effects) of the new medication.

In the present invention, only two dimensions (medication effects and medication side effects) are described for convenience of description. However, medication types may be determined on the basis of one dimension or more than two dimensions, and prescription effects and prescription side effects may be predicted on the basis of a medication type.

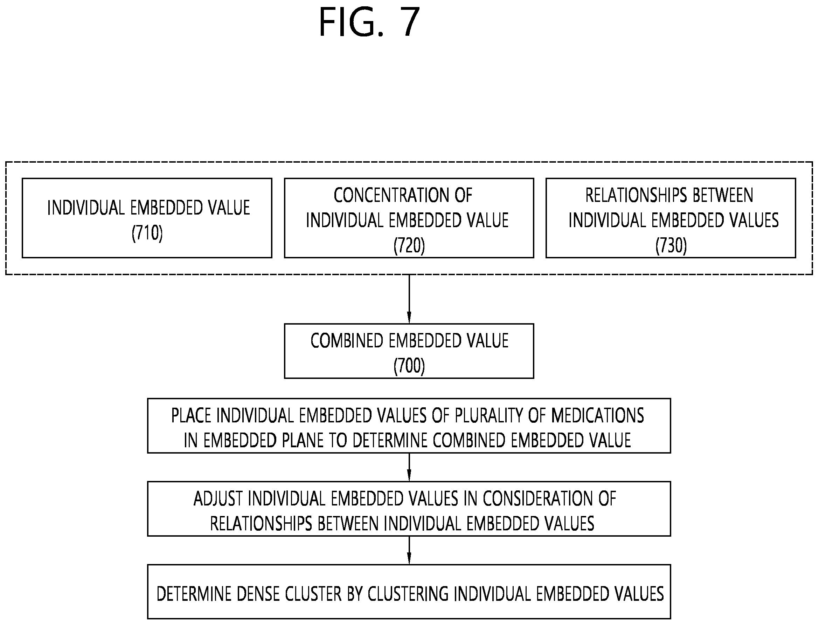

FIG. 7 is a conceptual diagram illustrating a method of generating a user-optimized artificial intelligence engine according to an exemplary embodiment of the present invention.

FIG. 7 shows a method of generating prescription data in consideration of a medication combination.

Referring to FIG. 7, a method of generating prescription data by predicting medication effects and medication side effects of a plurality of medication combinations is shown.

To consider medication effects and medication side effects of a combination of a plurality of medications, a combined embedded value 700 for a medication combination may be determined on the basis of individual embedded values 710 of the plurality of medications.

According to an exemplary embodiment of the present invention, the combined embedded value 700 may be generated on the basis of a combination of individual embedded values. Here, the combined embedded value 700 may be determined in consideration of the individual embedded values 710, concentration 720 of individual embedded values, and relationships 730 between individual embedded values.

First operation: The individual embedded values 710 of the plurality of medications may be positioned in an embedded plane to determine the combined embedded value 700. When a specific one of the individual embedded values 710 has a non-strong characteristic, that is, a threshold effect or less or a threshold side effect or less, the specific individual embedded value may be excluded.

Second operation: The individual embedded values 710 of the plurality of medications may be adjusted in consideration of whether effects are changed (e.g., reduced effects or enhanced effects) due to a combination of medications on the basis of the relationships 730 between individual embedded values or whether side effects are changed (e.g., reduced side effects or enhanced side effects) due to a combination of medications. For example, the individual embedded values 710 representing the degree of effects and the degree of side effects may be adjusted in consideration of the case where effects are enhanced, the case where side effects are reduced, and the like so that the positions in the embedded plane may be adjusted.

Third operation: The combined embedded value 700 may make clusters reflecting effects and side effects by clustering the individual embedded values 710. A dense cluster having a threshold concentration or more may be determined on the basis of concentrations of the individual embedded values 710 in the clusters. Clustering may be performed for each effect or each side effect. In other words, a clustering range is set to cluster the same or similar effects or the same or similar side effects so that a dense cluster may be determined among effects and among side effects

A combined embedded value may be finally determined on the basis of a concentration of individual embedded values in the dense cluster, and a prediction may be made on enhancement or reduction of a specific side effect and enhancement or reduction of a specific effect when a combination of a plurality of medications is taken. For example, when the concentration of a dense cluster corresponding to a specific effect is a threshold concentration or more, it is possible to predict enhancement of the effect, and when the concentration of a dense cluster corresponding to a specific side effect is the threshold concentration or more, it is possible to predict enhancement of the side effect.

According to exemplary embodiments of the present invention, enhancement or reduction of a specific side effect and enhancement or reduction of a specific effect caused by taking a combination of a plurality of medications can be predicted in consideration of changes of effects and side effects over time. For example, individual embedded values can be adjusted with reduction of effects over time, and individual embedded values can be adjusted with reduction of side effects over time. A user-optimized artificial intelligence engine can determine and provide the user with an intake routine for relatively enhancing effects and relatively reducing side effects in the case of taking a combination of a plurality of medications

The embodiments of the present invention described above may be implemented in the form of program instructions that can be executed through various computer units and recorded on computer readable media. The computer readable media may include program instructions, data files, data structures, or combinations thereof. The program instructions recorded on the computer readable media may be specially designed and prepared for the embodiments of the present invention or may be available instructions well known to those skilled in the field of computer software. Examples of the computer readable media include magnetic media such as a hard disk, a floppy disk, and a magnetic tape, optical media such as a compact disc read only memory (CD-ROM) and a digital video disc (DVD), magneto-optical media such as a floptical disk, and a hardware device, such as a ROM, a RAM, or a flash memory, that is specially made to store and execute the program instructions. Examples of the program instruction include machine code generated by a compiler and high-level language code that can be executed in a computer using an interpreter and the like. The hardware device may be configured as at least one software module in order to perform operations of embodiments of the present invention and vice versa.

While the present invention has been described with reference to specific details such as detailed components, specific embodiments and drawings, these are only examples to facilitate overall understanding of the present invention and the present invention is not limited thereto. It will be understood by those skilled in the art that various modifications and alterations may be made.

Therefore, the spirit and scope of the present invention are defined not by the detailed description of the present invention but by the appended claims, and encompass all modifications and equivalents that fall within the scope of the appended claims.

## Claims

1. A method of managing a prescription on the basis of a digital biomarker, the method comprising:
receiving, by a prescription management device, user data; and
generating, by the prescription management device, prescription data on the basis of the user data.

2. The method of claim 1, wherein the prescription management device generates the prescription data on the basis of an artificial intelligence engine,
wherein the artificial intelligence engine determines prescription effects and prescription side effects on the basis of the user data and generates the prescription data on the basis of the prescription effects and the prescription side effects.

3. The method of claim 2, wherein the artificial intelligence engine includes a default artificial intelligence engine and a user-optimized artificial intelligence engine,
wherein the user-optimized artificial intelligence engine is obtained by tuning the default artificial intelligence engine using the user data.

4. A prescription management apparatus for managing a prescription on the basis of a digital biomarker, the prescription management apparatus performing the operations of:
receiving user data; and
generating prescription data on the basis of the user data.

5. The prescription management apparatus of claim 4, wherein the prescription management apparatus generates the prescription data on the basis of an artificial intelligence engine,
wherein the artificial intelligence engine determines prescription effects and prescription side effects on the basis of the user data and generates the prescription data on the basis of the prescription effects and the prescription side effects.

6. The prescription management apparatus of claim 5, wherein the artificial intelligence engine includes a default artificial intelligence engine and a user-optimized artificial intelligence engine,
wherein the user-optimized artificial intelligence engine is obtained by tuning the default artificial intelligence engine using the user data.
